# EUROPEAN PATENT SPECIFICATION

(11) **EP 1 009 397 B1**
(45) Date of publication and mention of the grant of the patent: **23.04.2003**
(21) Application number: 98941201.0
(22) Date of filing: 03.09.1998
(51) Int. Cl.: A61K 31/165, A61P 29/00

(54) **USE OF N,N-BIS (PHENYLCARBAMOYLMETHYL) DIMETHYLAMMONIUM CHLORIDE AND DERIVATIVES IN THE TREATMENT OF CHRONIC PAIN**
VERWENDUNG VON N,N-BIS (PHENYLCARBAMOYLMETHYL) DIMETHYLAMMONIUMCHLORID UND DERIVATEN DAVON ZUR BEHANDLUNG VON CHRONISCHEM SCHMERZ
UTILISATION DE CHLORURE DE N,N-BIS (PHENYLCARBAMOYLMETHYL) DIMETHYLAMMONIUM ET SES DERIVES POUR LE TRAITEMENT DE LA DOULEUR CHRONIQUE

(30) Priority: 03.09.1997 US 56312 P
(43) Date of publication of application: 21.06.2000
(73) Proprietor: UCB Farchim S.A., CH-1630 Bulle (CH)
(72) Inventor: MACLEOD, Bernard, A., Vancouver, British Columbia V6R 2T4 (CA); QUASTEL, David, M., J., Vancouver, British Columbia V6J 4G5 (CA)
(74) Representative: Kirkham, Nicholas Andrew
(86) International application number: CA9800842
(87) International publication number: WO99011252

(56) References cited:
- NARAHASHI, TOSHIO ET AL: "Comparison of tertiary and quaternary amine local anesthetics in their ability to depress membrane ionic conductances" J. NEUROBIOL. (1972), 3(3), 267-76 CODEN: JNEUBZ, XP002093766
- VANDERHOEK, JACK Y. ET AL: "Local anesthetics, chlorpromazine and propranolol inhibit stimulus-activation of phospholipase A2 in human platelets" MOL. PHARMACOL. (1979), 16(1), 171-80 CODEN: MOPMA3;ISSN: 0026-895X, XP002093767
- NISHIZAWA, YUKIO ET AL: "Local anesthetics: stimulation of incorporation of inositol into phosphoinositides in guinea pig cerebral cortical synaptoneurosomes" BIOCHIM. BIOPHYS. ACTA (1990), 1054(2), 213-18 CODEN: BBACAQ;ISSN: 0006-3002, XP002093768
- WONG, KIN ET AL: "On the mechanisms of potentiation of local anesthetics by bicarbonate buffer: drug structure-activity studies on isolated peripheral nerve" ANESTH. ANALG. (N. Y.) (1993), 76(1), 131-43 CODEN: AACRAT;ISSN: 0003-2999, XP002093769
- FRAZIER, DONALD T. ET AL: "Site of action and active form of local anesthetics. II. Experiments with quaternary compounds" J. PHARMACOL. EXP. THER. (1970), 171(1), 45-51 CODEN: JPETAB, XP002093770
- VOLPI, M. ET AL: "Local anesthetics, mepacrine, and propranolol are antagonists of calmodulin" PROC. NATL. ACAD. SCI. U. S. A. (1981), 78(2), 795-9 CODEN: PNASA6;ISSN: 0027-8424, XP002093771
- KENDIG, JOAN J. ET AL: "Pressure antagonism to nerve conduction block by anesthetic agents" ANESTHESIOLOGY (1977), 47(1), 6-10 CODEN: ANESAV, XP002093772

## Description

### BACKGROUND OF THE INVENTION

At present three major classes of drugs are used to manage pain.

The first class of drugs consists of narcotic opiates, such as morphine, which block the perception of pain and act primarily in the brain and spinal cord. Members of this class of drugs are effective and potent but cause severe side effects such as sedation, constipation, and respiratory depression. These drugs are also known to cause severe addiction, which limits their usefulness in patients suffering from moderate pain.

The second class of drugs consists of local anesthetics which prevent pain in a specific region by blocking the transmission of signals through nerves. These drugs are given by injection to produce a high local concentration, often around specific sensory nerves to achieve nerve blocks. This type of pain control is practical only for interruption of acute pain and has little or no role in the management of chronic pain.

The third class of drugs consists of non-steroidal anti-inflammatory drugs, the most well known member of this class being aspirin. These drugs relieve pain by blocking the production or action of chemical mediators of pain and are used to control pain of low to moderate intensity.

In addition to the above, there are diverse drugs that have been found to be effective in the treatment of pain of particular etiology: this includes for example specific anti-migraine drugs.

Presently the only useful therapy for the treatment of chronic severe pain are the morphine-like drugs (Class I above) and since this use is limited in the majority of patients, there is an urgent and important need to identify new and alternate analgesic treatments.

Comparison of tertiary and quaternary amine local anesthetics in their ability to depress membrane ionic conductances is discussed in J. Neurobiology. Vol 3, No.3 pp.267-276.

### SUMMARY OF THE INVENTION

The present invention relates to the use of quaternary ammonium compounds for preparing a medicament for treating or preventing chronic pain. The compounds used in the present invention are certain quaternary ammonium compounds of Formula I, especially N, N-Bis (phenylcarbamoylmethyl) dimethylammonium chloride. It has now been found that these compounds offer potent and long-acting relief from pain when administered either systemically or locally. Furthermore, the side effects of the opiates or opiods (Class I drugs) mentioned above are not caused by the new therapy. The use of the present invention significantly improves the therapeutic alternatives for treating chronic pain and is particularly useful in the preventing and/or treating of subjects that have demonstrated erratic effects of conventional therapy. Since the new use also avoids the side effects of drugs used in currently available therapy, the present invention provides a safe, effective treatment of long duration for moderate to severe pain, such as for example neuropathic pain.

### DETAILED DESCRIPTION OF THE INVENTION

As used herein, the following terms have the following meaning:
"Alkyl" refers to a branched or unbranched hydrocarbon fragment containing the specified number of carbon atoms and having one point of attachment. Examples include n-propyl (A C₃ alkyl), isopropyl (also a C₃ alkyl) and t-butyl (a C₃ alkyl).
"Alkoxyalkyl" refers to an alkylene group substituted with an alkoxy group. For example, methyoxyethyl (CH₃OCH₂CH₂-) and ethoxymethyl (CH₃CH₂OCH₂-) are both C₃ alkoxyalkyl groups.
"Alkylene" refers to a divalent radical which is a branched or unbranched hydrocarbon fragment containing the specified number of carbon atoms and having two points of attachment. An example is propylene (-CH₂CH₂CH₂-), a C₃ alkylene.
"Aralkyl" refers to an alkylene group wherein one of the points of attachment is to an aryl group. An example is the benzyl group (C₆H₅CH₂-), a C₇ aralkyl group.
"Alkanoyloxy" refers to an ester substituent wherein the ether oxygen is the point of attachment to the molecule. Examples include propanoyloxy (CH₃CH₂C(=O)-O-), a C₃ alkanoyloxy and ethanoyloxy (CH₃C(=O)-O), a C₂ alkanoyloxy
"Alkoxy" refers to an O-atom substituted by an alkyl group, for example methoxy (-OCH₃), a C₁ alkoxy.
"Alkoxycarbonyl" refers to an ester substituent wherein the carbonyl carbon is the point of attachment to the molecule. Examples include ethoxycarbonyl (CH₃CH₂OC=O), a C₃ alkoxycarbonyl, and methoxycarbonyl (CH₃OC(=O)-)₁ a C₂ alkoxycarbonyl.
"Aryl" refers to aromatic groups which have at least one ring having a conjugated pi electron system and includes carbocyclic aryl, heterocyclic aryl (also known as heteroaryl groups) and biaryl groups, all of which may be optionally substituted. Carbocyclic aryl groups are generally preferred in the compounds of the present invention, wherein phenyl and naphthyl groups are preferred carbocyclic aryl groups.
"Cycloalkyl" refers to a ring, which may be saturated or unsaturated and monocyclic, bicyclic or tricyclic formed entirely from carbon atoms. An example is the cyclopentenyl group (C₅H₇-), which is a five carbon unsaturated cycloalkyl group.
"Carbocyclic" refers to a ring which may be either an aryl ring or a cycloalkyl ring, both as defined above.
"Thioalkyl" refers to a sulfur atom substituted by an alkyl group, for example thiomethyl (CH₃S-), a C₁ thioalkyl.

The present invention provides a sustained and effective treatment and/or prevention of pain offered by pharmaceutical compositions comprising one or more of the compounds encompassed within Formula I. This is of significant therapeutic importance, since many patients taking conventional pain medication experience serious side effects as well as erratic therapeutic effects of their medication, particularly in long-term use of the same. The finding that compounds encompassed within Formula I offer an effective and prolonged therapeutic activity against pain when given either orally or parenterally is unexpected and surprising, given the fact that although compounds encompassed within this formula have chemical similarities with some local anesthetics, the latter compounds do not generally share the analgesic activity of the compounds used in the invention.

in contrast to the opiates and opiods, the compounds of Formula I do not appear to cause any significant modification to the perception of pain; in contrast to the nerve-blocking activity of local anesthetics; the compounds of Formula I do not indiscriminately block nerve conduction and in contrast to the major compounds of Class 3, are not potent cyclooxygenase inhibitors. Thus, the potent pain inhibition offered by the compounds used in the present invention is both surprising and unexpected.

This finding is of significant therapeutic and toxicological importance since the activity of the pain treatment is improved with the invention, and the conventional toxicity of pain medication will be largely avoided by treatment of the patient using the medicament manufactured according to the present invention.

Formula I is represented by the following formula: 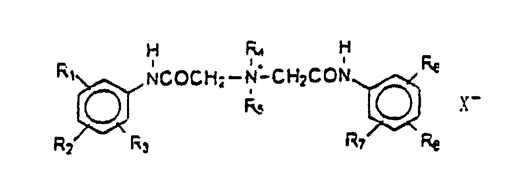 wherein R₁, R₂, R₃, R₆, R₇ and R₈ are independently selected from bromine, chlorine, fluorine, carboxy, hydrogen, hydroxy, hydroxymethyl, methanesulfonamido, nitro, sulfamyl, trifluoromethyl, C₂-C₇-alkanoyloxy, C₁-C₆-alkyl, C₁-C₆-alkoxy, C₂- C₇- alkoxycarbonyl, N(R₉,R₁₀), phenyl and C₁-C₆-thioalkyl; and where R₉ and R₁₀ are independently hydrogen, acetyl, methanesulfonyl, or C₁-C₆-alkyl; R₄ and R₅ are independently selected from hydrogen, C₁-C₈ alkyl, C₃-₈ alkoxyalkyl and C₇-C₁₂ aralkyl: X is the anion of a pharmaceutically acceptable salt; and isolated enantiomeric. diasteriometic and geometric isomers thereof, and mixtures thereof.

A preferred compound for use in the present invention is a compound of Formula I wherein R₁, R₂, R₃, R₆, R₇ and R₈ are each hydrogen, R₄ and R₅ are each methyl, and X⁻ is chloride anion. This compound ("Compund I") was synthesized as described in Belgium Patent No. 614,154, by Traunt and Dahlborn, 1962, which follows the Swedish patent 1779/61, (see also T. Takahashi, J. Okada, M. Hori, A. Kato, K. Kanematsu, and Y. Yamamoto. *J*. *Pharm. Soc. Japan* **76,** 1180-6 (1956)). A conventional route for synthesis involves three (3) steps and can be described (as in the aforementioned patent) as follows:

### i) Chloroacetanilide

To a chilled solution of aniline (37.2 g, 0.40 mol) and potassium carbonate (66.4 g, 0.48 mol) in chloroform (200 ml) was added dropwise via cannula a solution of chloroacerylchloride (49.6 g, 0.44 mol) in chloroform (100 ml) and the reaction mixture was heated to 55°C for 90 minutes. To the cooled reaction mixture was then added water (300 ml), the organic layer was collected and the aqueous layer was extracted twice more with chloroform (2 x 100 ml). The combined organic layers were dried over sodium sulfate and evaporation of the solvent in vacuo provided the crude product. The product was purified via extraction through a Soxhlet apparatus with diethyl ether to provide 22.7 g of the desired chloroacetanilide. m.p. 133-135°C. ¹H NMR (CDC1₃, 200 MHz) δ : 8.5 (br. s, NH, 1H), 7.6-7.1 (m, Ar, 5H) 4.1 (s, CH₂, 2H).

### ii) Dimethylaminoacetanilide

A mixture of chloroacetanilide (10.0 g, 59 mmol) in dimethylamine, 40% wt in water (100 ml) was refluxed for 4 hour. The cooled reaction mixture was partitioned between dichloromethane (100 ml) and 1M NaOH aqueous solution (100 ml). The aqueous layer was extracted twice more with dichloromethane (2 x 100 ml), the combined organic layers were concentrated in vacuo to a volume of approximately 100 ml and washed with water (2 x 100 ml) in order to remove the remaining dimethylamine. The organic layer was collected, dried over sodium sulfate and the solvent evaporated in vacuo to provide 10.2 g (97% yield) of the pure dimethylaminoacetanilide. ¹H NMR (CDC1₃, 200 MHz) δ : 9.1 (br. s, NH, IH), 7.6-7.0 (m, Ar, 5H) 3.1 (s, CH₂, 2H), 2.4 (s CH₃, 6H)

### iii) N,N-Bis- (phenylcarbamoylmethyl) dimethylainmonium chloride

A mixture of chloroacetanilide (10.1g, 59.5 mmol), dimethylaminoacetanilide (10.7 g, 60 mmol) and potassium iodide, 99+% (0.1 g, 0.6 mmol) in dry xylene (30 ml) was refluxed for 1 hour and then allowed to stand overnight to ambient temperature. The solvent was decanted and the remaining gummy solid was triturated in diethyl ether in order to obtain a whitish powder. The resulting solid was collected and recrystallized in a mixture of ethanol and diethyl ether to provide 9.3 g (45% yield) of the desired ammonium salt. m.p. 177-178°C, ¹H NMR (DMSO-d₆, 300 MHz) δ:11.3 (s, NH, 2H), 7.7-7.1 (m, Ar, 10 H) 4.8 (s, CH₂, 4H) , 3.6 (s CH₃, 6H), ¹³C NMR (DMSO-d_{6,} 75 MHz) δ:162.1(+), 137.8 (+), 128.8 (-), 124.3 (-), 119.7 (-), 63.0 (+), 52.8 (-), LRMS(EI) m/z=297 (0.95%, M⁺⁻CH3), elemental analysis calculated for C₁₈H₂₂N₄O₂C1 (347.84): C, 62.15; H, 6.37; N, 12.08; found C, 61.75; H, 6.50; N, 12.04.
Reference: A.P. Traunt and J.R. Dahlbom Belgium Patent No. 614154, Feb. 20, 1961.

### Synthetic Scheme:

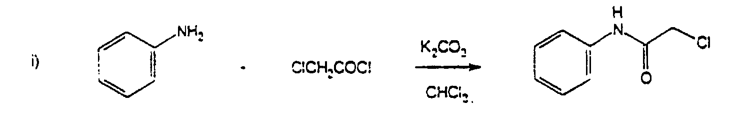

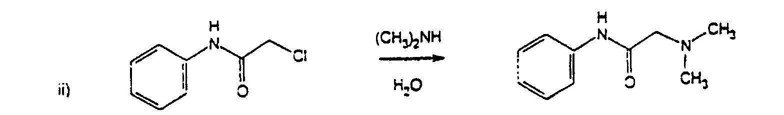

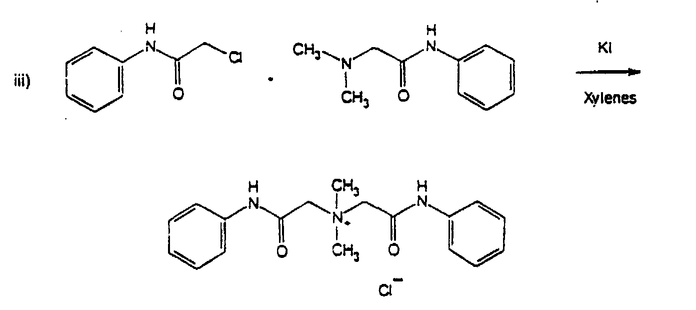

Other compounds encompassed by Formula I can be synthesized in an analogous manner and are within the skill in the art. A typical synthetic scheme is as follow:

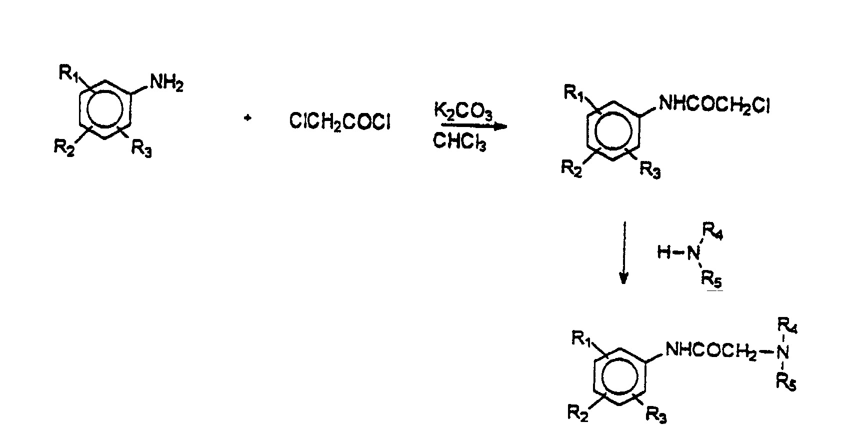

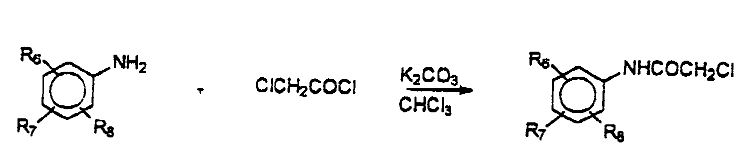

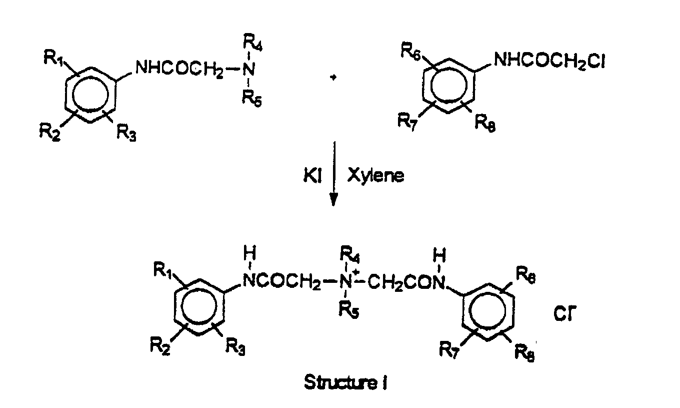

Suitable pharmaceutically acceptable salts include acid addition salts of acids such as hydrochloric, hydrobromic, benzenesulfonic (besylate), benzoic, camphorsulfonic, ethanesulfonic, fumaric, gluconic, glutamic, isethionic, maleic, malic, mandelic, methanesulfonic, mucic, nitric, parnoic, pantothenic, succinic, p-toluenesulfonic, phosphoric, sulphuric, citric, tartaric, lactic and acetic acid, although the preferred acid addition salt is the hydrochloride salt.

The magnitude of the prophylactic or therapeutic dose of the compounds used in the present invention in the chronic management of pain will vary with the severity and nature of the condition being treated and the route of administration. The dose and the frequency of the dosing will also vary according to age, body weight and response of the individual patiem. In general, the total daily dose range for the compounds of the present for the conditions described herein is from about 10 mg to about 20 mg in single or repeated doses, preferably in repeated doses. In managing the patient, the therapy should be initiated at a lower dose, perhaps at about 10 mg to about 50 mg, and may be increased up to about 200 mg depending on the patient's global response. For pharmacokinetic reasons, it may also be preferred to administer an initial loading (bolus) dose of the drug to patients suffering from pain. It is further recommended that patients be titrated, based on individual response(s). It may be necessary to use dosages outside these ranges, as will be apparent to those skilled in the art. Further, it is noted that the clinician or treating physician will know how and when to interrupt, adjust or terminate therapy in conjunction with individual patient response. The terms "a therapeutically effective amount" and "an amount sufficient to treat pain syndrome but insufficient to cause adverse effects" are encompassed by the above-described dosage amounts and dose-frequency schedule.

Any suitable route of administration may be employed for providing the patient with an effective dosage of the compounds used in the present invention. For example, oral, sublingual, rectal, parental (subcutaneous, intramuscular, intravenous, etc.), transdermal, topical and like forms of administration may be employed. Dosage forms include but are not limited to solid dosage forms, suspensions, solutions, creams, gels or eiixers. For example, tablets, troches, dispersions, suspensions, solutions, capsules, microencapsulated systems, sprays; topical delivery systems, and the like are suitable. Because of their ease of administration, tablets and capsules represent some of the more advantageous oral dosage unit forms, in which case solid pharmaceutical carriers are employed. If desired, tablets may be coated by standard aqueous or nonaqueous techniques.

In addition to the common dosage forms set forth above, the compounds used in the present invention also may be administered by controlled release means and delivery devices such as those described in U.S. Patent Nos. 3,845,770, 3,916,899, 3,536,809, 3,598,123 and 4,008,719, and PCT application 1) 92/20377, the disclosures of which are hereby incorporated by reference.

Pharmaceutical compositions mode according to the present invention suitable for oral administration may be presented as discrete unit dosage forms such as capsules, cachets or tablets, each containing a predetermined amount of the active ingredient, as a powder or granules, or as a solution or a suspension in an aqueous liquid, a nonaqueous liquid, an oil-in-water emulsion, or a water-in-oil liquid emulsion. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent1 surface active agent or dispersing agent. Molded tablets may be made by molding, in a suitable machine, a mixture of the powdered compound moistened with an inert liquid diluent, All of the foregoing techniques are well known to persons of skill in the pharmaceutical art. Each tablet may contain from about 5 mg to about 200 mg of the active ingredient.

The pharmaceutical compositions made according to the present invention comprise the compounds used in the present invention as the active ingredient, including pharmaceutically acceptable salts thereof, and may also contain a pharmaceutically acceptable carrier, and optionally other therapeutic ingredients and conventional additives, including aqueous based carriers, co-solvents such as ethyl alcohol, propylene glycol and glycerin, fillers, lubricants, werting agents, flavoring agents, coloring agents, emulsifying, suspending or dispersing agents, suspending agents, etc.

The terms "pharmaceutically acceptable salts" or "a pharmaceutically acceptable salt thereof" refer to salts prepared from pharmaceutically acceptable non-toxic acids. Suitable pharmaceutically acceptable acid addition salts for the compound used in the present invention include acetic, benzene sulfonic (besylate), benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, parnoic, pantothenic, phosphoric, succinic, sulfuric, tartaric, p-toluenesulfonic, and the like. The hydrochloride salt is particularly preferred.

In the use of the present invention, the compounds can be administered together with one or more other compound(s), most often a conventional pain medication belonging to any of the classes mentioned above. Compounds that improve or prolong the therapeutic effect of the compounds used in the present invention, e.g., compounds that inhibit the metabolic or chemical degradation of the compounds used in the present invention, may also be co-administered to patients. The two (or more) drugs (compounds of Formula I and one or more other drugs) can be administered in one composition or as separate entities. For example, they can be administered in a single formulation, such as a capsule; tablet, powder, or liquid, mist aerosol, infection, etc. or as separate formulations. The components included in a particular formulation, in addition to the compound(s) of Formula I and another drug or drugs, are determined primarily by the manner in which the composition is to be administered. For example, a composition to be administered in tablet form can include a filler (e.g., lactose), a binder (e.g., carboxymethyl cellulose, gum arabic, gelatin), an adjuvant, a flavoring agent. a coloring agent and a coating material (e.g., wax or a plasticizer). A composition to be administered in liquid form can include the combination of drugs and, optionally, an emulsifying agent. a flavoring agent and/or a coloring agent. A composition to be administered rectally may include the combination of drugs consisting of one or more compounds used in the present invention and for example at least one additional drug selected from the group consisting of analgesics, local anesthetics, antinistamines, antiserotonergics, metabolic inhibitors, and other agents with synergistic pharmacological efficacy.

In general, according to the use of the present invention. one or more of the compounds of Formula I, alone or in combination with another drug or drugs, is administered to an individual suffering from pain, periodically or continuously, as necessary to manage or eliminate pain and to improve the quality of life.

Examples of suitable administration for various types of pain are as follows:

### (a) Local application.

(1) Pain consequent to joint surgery may be averted by intraarticular injections of a pharmaceutically acceptable composition containing one or more compounds of Formula I in concentrations of 0.1% to 5%.
(2) Arthritic pain or pain of bursitis will successfully be treated by similar injections as above.
(3) Pain of burns also can be successfully treated since the degree of analgesia offered by the compounds of Formula I will block the excruciating pain associated with dressing changes.
(4) Pain from an eye infection or trauma will successfully be treated by similar applications of the compounds

### (b) Systemic administration

Systemic administration of the compounds used in the present invention generally results in analgesia without the adverse side effects known for analgesic compounds of the Classes 1, 2 and 3, as stated above. This type of administration may be particularly useful in the treatment andlor prevention of pain associated with cancer. diabetes and a variety of neurological diseases as well as pain associated with bums, surgery, and trauma. It may be particularly useful in the treatment of patients suffering from various neuropathic pain syndromes.

### (c) Intravenous regional anesthesia of the extremities

Intravenous regional analgesia of limbs in surgery may be achieved with the compounds used in the present invention, its advantage over presently used local anesthetics being the absence of central nervous system toxicity and the other side effects when the drug is released into the circulation after the completion of surgery. The "release toxicity" of local anesthetics limits the use of regional anesthesia to upper limb surgery, a constraint that does not apply with the use of the analgesic compound used in this invention.

The following examples indicate the therapeutic usefulness of the compounds used according to the present invention, although the present invention is not limited to these examples.

### EXAMPLE 1

The compound used in the present invention is the compound of Formula I wherein R₁, R₂, R₃, R₆, R₇ and R₈ are each hydrogen, R₄ and R₅ are each methyl, and X is chloride anion ("Compound I").

### ORAL UNIT DOSAGE FORMULATION

| Tablets Ingredients | per tablet | per batch of 10,000 tablets |
|---|---|---|
| Compound I | 25 mg | 250 g |
| Microcrystalline cellulose | 30 mg | 300 g |
| Lactose | 70 mg | 700 g |
| Calcium stearate | 2 mg | 20 g |
| FD&C Blue # Lake | 0.03 mg | 300 mg |

The selected compound is blended with the lactose and cellulose until a uniform blend is formed. The lake is added and further blended. Finally, the calcium stearate is blended in, and the resulting mixture is compressed into tablets using a 9/32 inch (7 mm) shallow concave punch. Tablets of other strengths may be prepared by altering the ratio of active ingredient to the excipients or to the final weight of the tablet.

The surprising utility of the compounds used according to the present invention has been established by the following studies.

### EXAMPLE 2

### 1. Acute Toxicity in Mice, Rats and Rabbits

The experiments were carried out on animals that were administered intravenously or orally escalating doses of the test compounds. After administration of Compound I, the survival dose values (LD₅₀) were 125 mg/kg after intraperitoneal administration to mice, 264 mg/kg after subcutaneous injection to mice, 150 mg/kg after intraperitoneal administration to rats, 117 mg/kg after oral administration to mice and 17 mg/kg after intravenous administration to rabbits.

Toxicity (LD₁₀₀) in other mammals was reported by Marchetti et al. to be 36 mg/kg in guinea pig after intravenous administration and 50 mg/kg in rabbit after intravenous administration. (G. Marchetti, L. Merlo, L. Lombardi and M. Cicardi, Arch. Ital. Sci. Farmacol. 14(1), 33-45 (1964)).

### 2. Systemic Analgesic Effects

The analgesic efficacy of compounds of Formula I was established by the following studies.

### 2.1. Formalin Paw Test (D. Dubuisson and S.J. Dennis, Pain, 1977, 4 161 174)

In this test a hind paw (footpad) of conscious mice was injected with a formalin solution, which produced a painful stimulus, to which the mice reacted by licking the foot. Pretreatment of the animal with an effective analgesia reduced the pain and consequently the licking behavior.

The method to demonstrate the analgesic effects of Compound I is briefly as follows. At t=0 minutes, either saline, 70, 20, 40, 80 or 160 mg/kg was injected subcutaneously under the dorsal skin of the neck of a CD-I mouse (n=8 for each dose of Compound I) that weighed in the range of 20-45 grams. At t=60 minutes, formalin (2 different amounts were used in separate studies) was injected into the right hind paw (footpad) of each mouse, and the animal was released into the observation chamber (24-26°C). The mice were videotaped in groups of four for 60 minutes. The observations were made from viewing the videotapes activity, and the investigators looked for the following pain behavioral traits: licking of the right hind paw; lifting and licking of the right hind paw; resting/sleeping/being still; exploring or grooming (F.V. Abbott, K.B.J. Franklin and R.F. Wesbrook, Pain, 1995, 60 191-202). The corresponding behavioral traits were checked and recorded for each animal every two minutes for sixty minutes after the formalin injection.

Results showed Compound I produced a dose related inhibition of the delayed phase of licking, which is indicative of pain relief.

In the study where 30µ1 of 2.5% formalin was injected, the ED₅₀ was determined to be 145 mg/kg. In the study where 20 µ1 of 2.0% formalin was injected, the ED₅₀ was determined to be 50 mg/kg and more than 80% inhibition was obtained at 160 mg/kg of Compound I. At these doses there was no evident alteration of animal behavior other than the reduced reaction to the noxious stimulus.

### 2.2. Formalin Tail Test

The protocol of this test is similar to that for the Paw Test described above, except that the injection of formalin was in the dorsal mid-tail region of the mouse instead of the hind paw. Behaviors such as licking of the tail (with and without lifting the tail), grooming, exploring and resting were recorded every two minutes for sixty minutes after injection of formalin (30 µ1 of 2.5% formalin)

Two modes of administration (subcutaneous and oral) of Compound I were studied. Subcutaneous administration of Compound I under the dorsal skin of the neck produced a dose related inhibition of the delayed phase of licking which is indicative of pain relief. The ED₅₀ was determined to be 40 mg/kg.

In the oral administration study, CD-1 mice weighing 15-30 grams each (n=8 or 4 for each dose of Compound I) were orally administered with either gum arabic (vehicle), 300 mg/kg or 700 mg/kg Compound I, 60 minutes prior to formalin (2.5%, 30 µ1)_injection. Behaviors such as licking of the tail (with and without lifting the tail), grooming, exploring and resting were recorded every two minutes for sixty minutes after injection of formalin (30 µ1 of 2.5% formalin). At an oral dose of 700mg/kg, Compound I reduced licking by 62%, while at 300 mg/kg, a reduction of 50% was determined

### 3. Regional Analgesic Effects in Mice

In these experiments a tourniquet is placed around the base of a mouse tail so as to occlude blood flow to and from the tail. Injection of Compound I into the tail vein then rapidly produced block of tail-flick in response to pin-prick but not to heat, which indicates a selective analgesic activity of Compound I. In parallel experiments, lidocaine inhibits responses both to pin-prick and to heat, which indicates a local anesthetic effect of that drug.

4. In experiments using intradermal injections into the human forearm of small volumes of solutions containing Compound I, the drug was found to cause a long-lasting inhibition of pain induced by pin-prick, with little or no effect on sensation of heat or touch, which indicates a selective analgesic activity of the Compound I. Intradermal injections of local anesthetic compounds (examples include lidocaine and bupivacaine) inhibited equally sensation of pain, heat and touch.

## Claims

1. Use of a compound for the manufacture of a medicament for treating or preventing chronic/non-acute pain in warm blooded animals including humans, the compound having the following formula: 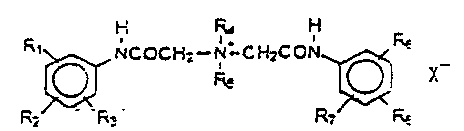 wherein R₁, R₂, R₃, R₆, R₇ and R₈ are independently selected from bromine, chlorine, fluorine, carboxy, hydrogen, hydroxy, hydroxymethyl, methanesulfonamido, nitro, sulfamyl, trifluoromethyl, C₂-C₇-alkanoyloxy, C₁-C₆-alkyl, C₁-C₆-alkoxy, C₂-C₇-alkoxycarbonyl, N(R₉,R₁₀), phenyl and C₁-C₆-thioalkyl; and where R₉ and R₁₀ are independently hydrogen, acetyl, methanesulfonyl, or C₁-C₆-alkyl; R₄ and R₅ are independently selected from hydrogen, C₁-C₈ alkyl, C₃-C₈ alkoxyalkyl and C₇-C₁₂ aralkyl; X⁻ is the anion of a pharmaceutically acceptable acid; or isolated enantiomeric, disasteriomeric and geometric isomers thereof, or mixtures thereof.

2. The use of claim 1, wherein R₁, R₂, R₃, R₆, R₇ and R₈ are each hydrogen, R₄ and R₅ are each methyl, and X⁻ is chloride anion.

3. The use of claim 1 or claim 2, presented in a form to be administered parenterally, transdermally, rectally, topically or orally.

4. The use of claim 1, presented in a form to be administered orally in an amount of from about 1 to about 250mg once or up to four times daily, or in a form to be administered parenterally in a solution from about 0.1% to about 5% once or up to four times daily.

5. The use according to any one of claims 1 to 4, wherein the chronic/non-acute pain is selected from the group consisting of pain after joint surgery, arthritis, bursitis, eye infection, eye trauma, burns, cancer, diabetes and neurological disorders.

## Patentansprüche

1. Verwendung einer Verbindung der folgenden Formel zur Herstellung eines Arzneimittels zur Behandlung oder Vorbeugung von chronischem/ nicht akutem Schmerz in warmblütigen Tieren, einschließlich Menschen 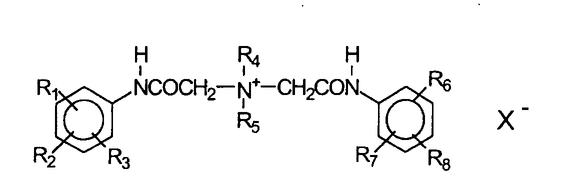 worin R₁, R₂, R₃, R₆, R₇ und R₈ unabhängig voneinander ausgewählt sind aus Brom, Chlor, Fluor, Carboxy, Wasserstoff, Hydroxy, Hydroxymethyl, Methansulfonamido, Nitro, Sulfamyl, Trifluormethyl, C₂-C₇-Alkanoyloxy, C₁-C₆-Alkyl, C₁-C₆-Alkoxy, C₂-C₇-Alkoxycarbonyl, N(R₉,R₁₀), Phenyl und C₁-C₆-Thioalkyl, und worin R₉ und R₁₀ unabhängig voneinander Wasserstoff, Acetyl, Methansulfonyl oder C₁-C₆-Alkyl sind, R₄ und R₅ unabhängig voneinander ausgewählt sind aus Wasserstoff, C₁-C₈-Alkyl, C₃-C₈-Alkoxyalkyl und C₇-C₁₂ Aralkyl, X⁻ das Anion einer pharmazeutisch verträglichen Säure ist, oder ihrer isolierten Enantiomeren, Diastereomeren und geometrischen Isomeren, oder Mischungen davon.

2. Verwendung nach Anspruch 1, worin R₁, R₂, R₃, R₆, R₇ und R₈ jeweils Wasserstoff sind, R₄ und R₅ jeweils Methyl sind und X⁻ ein Chloridanion ist.

3. Verwendung nach Anspruch 1 oder 2, dargeboten in einer Form zur parenteralen, transdermalen, rektalen, topischen oder oralen Verabreichung.

4. Verwendung nach Anspruch 1, dargeboten in einer Form zur oralen Verabreichung in einer Menge von etwa 1 bis etwa 250 mg einmalig oder bis zu viermal täglich, oder in einer Form zur parenteralen Verabreichung in einer Lösung von etwa 0,1% bis etwa 5% einmalig oder oder bis zu viermal täglich.

5. Verwendung nach Anspruch 1 bis 4, worin der chronische/nicht-akute Schmerz ausgewählt ist aus der Gruppe bestehend aus dem Schmerz nach einer Gelenkoperation, Arthritis, Bursitis, Augenentzündung, Augenverletzung, Verbrennungen, Krebs, Diabetes und neurologischen Störungen.

## Revendications

1. Utilisation d'un composé pour la fabrication d'un médicament pour le traitement ou la prévention d'une douleur chronique / non aiguë chez les animaux à sang chaud comprenant les êtres humains, le composé ayant la formule suivante: 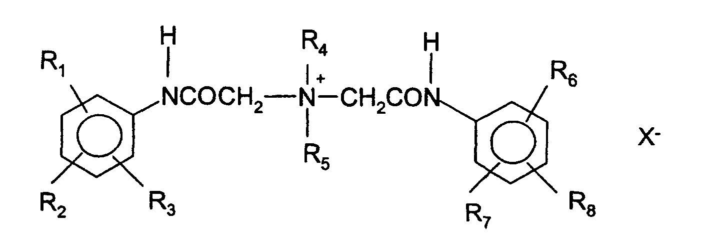 dans laquelle R₁, R₂, R₃, R₆, R₇ et R₈ sont choisis indépendamment parmi un atome de brome, un atome de chlore, un atome de fluor, un groupe carboxy, un atome d'hdrogène, un groupe hydroxy, un groupe hydroxyméthyle, un méthanesulfonamide, un groupe nitro, un groupe sulfamyle, un groupe trifluorométhyle, un groupe alcanoyloxy en C₂ à C₇, un groupe alkyle en C₁ à C₆, un groupe alcoxy en C₁ à C₆, un groupe alcoxycarbonyle en C₂ à C₇, N(R₉,R₁₀), un groupe phényle et un groupe thioalkyle en C₁ à C₆ ; et où R₉ et R₁₀ sont, indépendamment, un atome d'hydrogène, un groupe acétyle, un groupe méthansulfonyle, ou un groupe alkyle en C₃ à C₆ ; R₄ et R₅ sont indépendamment choisis parmi un atome d'hydrogène, un groupe alkyle en C₁ à C₈, un groupe alcoxyalkyle en C₃ à C₈ et un groupe aralkyle en C₇ à C₁₂ ; X⁻ est l'anion d'un acide pharmaceutiquement acceptable ; ou des isomères énantiomériques, diastéréoisomériques et géométriques de celui-ci, ou des mélanges de ceux-ci.

2. Utilisation selon la revendication 1, dans laquelle R₁, R₂, R₃, R₆, R₇ et R₈ sont chacun un atome d'hydrogène, R₄ et R₅ sont chacun un groupe méthyle, et X⁻ est un anion chlorure.

3. Utilisation selon la revendication 1 ou la revendication 3, présentée sous une forme à administrer par voie parentérale, transdermique, rectale, topique ou orale.

4. Utilisation selon la revendication 1, présentée sous une forme à administrer par voie orale en une quantité comprise dans la plage allant d'environ 1 à environ 250 mg une fois ou jusqu'à quatre fois par jour, ou sous une forme à administrer par voie parentérale en une solution d'environ 0,1 % à environ 5 % une fois ou jusqu'à quatre fois par jour.

5. Utilisation selon l'une quelconque des revendications 1 à 4, dans laquelle la douleur chronique / non aiguë est choisie dans le groupe constitué des douleurs articulaires post-chirurgicales, de l'arthrite, de la bursite, des infections oculaires, des traumatismes oculaires, des brûlures, du cancer, du diabète et des troubles neurologiques.
